# EUROPÄISCHE PATENTSCHRIFT

(11) **EP 0 648 331 B1**
(45) Veröffentlichungstag und Bekanntmachung des Hinweises auf die Patenterteilung: **24.04.1996**
(21) Anmeldenummer: 93914590.0
(22) Anmeldetag: 21.06.1993
(51) Int. Cl.: G01N 33/00

(54) **VERFAHREN UND VORRICHTUNG ZUR ERMITTLUNG EINES GEMISCHANTEILS EINES GASGEMISCHES**
PROCESS AND DEVICE FOR DETERMINING A FRACTION OF A GAS MIXTURE
PROCEDE ET DISPOSITIF PERMETTANT DE DETERMINER UNE PARTIE D'UN MELANGE GAZEUX

(30) Priorität: 02.07.1992 DE 4221692
(43) Veröffentlichungstag der Anmeldung: 19.04.1995
(73) Patentinhaber: SIEMENS AKTIENGESELLSCHAFT, D-80333 München (DE)
(72) Erfinder: ECKARDT, Bernd, D-6454 Bruchköbel (DE)
(86) Internationale Anmeldenummer: DE9300531
(87) Internationale Veröffentlichungsnummer: WO9401767

(56) Entgegenhaltungen:
- DE-A- 3 438 659
- GB-A- 2 153 073
- US-A- 4 072 043
- US-A- 4 361 028

## Beschreibung

Die Erfindung bezieht sich auf ein Verfahren zur Ermittlung eines Gemischanteils eines Gasgemisches in der Containment-Atmosphäre eines Kernkraftwerks, durch Messung der bei einer katalytischen Reaktion entstehenden Temperaturänderung. Sie bezieht sich weiter auf eine Vorrichtung zur Durchführung des Verfahrens.

Es ist bekannt, daß Wasserstoff in Anwesenheit eines Katalysators, z.B. auf Platin- oder Palladiumbasis, schon bei Raumtemperatur in einer exothermen Reaktion oxidiert wird. Diese katalytische Oxidation von Wasserstoff wird auch als kalte Verbrennung bezeichnet. Durch Messung der bei dieser Reaktion entstehenden Wärmetönung oder Temperaturänderung kann die Wasserstoffkonzentration in einem Gasgemisch ermittelt werden.

So wird z.B. bei einem aus der US-PS 4,298,574 bekannten Verfahren die infolge einer katalytischen Oxidation von Wasserstoff an einem Katalysator entstehende Temperaturänderung in bezug auf einen Referenzwert mittels eines Thermoelementes erfaßt und in ein entsprechendes Spannungssignal umgewandelt. Die gemessene Spannung ist ein Maß für den Wasserstoffanteil des Gasgemisches. Mit diesem Verfahren kann auch der Anteil an Kohlenmonoxid oder Kohlenwasserstoff im Gasgemisch ermittelt werden.

Bei einem aus der DE-OS 30 46 560 bekannten Verfahren zur Feststell g von brennbaren Gasen, insbesondere von Wasserstoff in der Containment-Atmosphäre eines Kernkraftwerks, wird eine Temperaturänderung mittels eines temperaturabhängigen ohmschen Widerstands erfaßt, der Teil einer Brückenschaltung ist.

Um ein reaktionsfähiges Wasserstoff/Sauerstoff-Gemisch zur Detektion von Wasserstoff in einem inerten Gasstrom zu erhalten, wird bei einem aus der US-PS 4,072,043 bekannten Verfahren dem inerten Gas (z.B. N₂) vor dessen Eintritt in eine Detektionskammer Luft zugemischt.

Bei den bekannten Verfahren besteht allerdings die Gefahr, daß sich der Katalysator bis an die Zündgrenze eines zündfähigen Gemisches erhitzt. Dies kann aufgrund der hohen Wärmeentwicklung zu einer Zerstörung des Katalysators führen. In der Containment-Atmosphäre eines Kernkraftwerks können sich zündfähige Gemische bereits bei einem Wasserstoffanteil von weniger als 10% unkontrolliert mit hohen Raten abbauen, wobei dann eine zuverlässige Ermittlung der Gemischanteile nicht mehr möglich ist.

Um auch bei einer über einen Grenzwert hinaus ansteigenden Konzentration eines brennbaren Gases innerhalb einer Gasprobe dennoch eine zufriedenstellende Detektion des brennbaren Gases zu ermöglichen, ist es aus der GB-OS 2 153 073 bekannt, ein Verdünnungsmittel, insbesondere Umgebungsluft, in eine die Gasprobe führende Leitung zu pumpen.

Der Erfindung liegt daher die Aufgabe zugrunde, ein Verfahren der eingangs genannten Art derart weiterzubilden, daß eine zuverlässige Ermittlung eines Gemischanteils, insbesondere des Wasserstoff- und/oder Sauerstoffanteils, eines Gasgemisches in der Containment-Atmosphäre eines Kernkraftwerks, über einen großen Bereich möglich ist. Weiter soll eine besonders einfache Vorrichtung zur Durchführung des Verfahrens angegeben werden.

Bezüglich des Verfahrens der eingangs genannten Art wird diese Aufgabe erfindungsgemäß durch die Kennzeichnenden Merkmale des Ansprüchs 1 gelöst.

Das dem Katalysator zugeführte Gasgemisch wird mit einem Treibgas bekannter Zusammensetzung verdünnt. Die Verdünnung erfolgt in einer Strahlpumpe, z.B. einer Venturidüse. Dazu wird das Gasgemisch zweckmäßigerweise über einen Filter angesaugt und mit dem Treibgas oder einem Treibgasgemisch verdüst. Dabei ist die Verdünnung einstellbar, vorzugsweise im Verhältnis 1 : 1 bis 1 : 10, z.B. im Verhältnis 1 : 4.

Zur Ermittlung des Wasserstoff- oder Kohlenmonoxidanteils im Gasgemisch kann das Treibgas auch einen oxidierenden Anteil, vorzugsweise Sauerstoff, enthalten. Als Treibgas kann dann entweder Luft oder, wenn genügend Sauerstoff im Gasgemisch enthalten ist, auch Stickstoff verwendet werden.

Zur Ermittlung des Sauerstoffanteils im Gasgemisch enthält das Treibgas zweckmäßigerweise einen oxidierbaren Anteil, z.B. Wasserstoff oder Kohlenmonoxid.

Bezüglich der Vorrichtung zur Dürchführung des Verfahrens mit Mitteln zur Messung der bei einer katalytischen Reaktion entstehenden Temperaturänderung wird die genannte Aufgabe erfindungsgemäß gelöst durch die Kennzeichnenden Merkmale des Ansprüchs 7.

Die zur Verdünnung des Gasgemisches mit dem Treibgas zweckmäßigerweise eingesetzte Strahlpumpe mündet vorteilhafterweise direkt in den Katalysator. Sie kann dann zusammen mit dem Katalysator in einem Gehäuse angeordnet sein, wobei ihr ein separates Filter vorgeschaltet sein kann. Alternativ kann die Strahlpumpe aber auch über eine Leitung mit dem Katalysator verbunden sein. Sie ist dann in einem separaten Gehäuse angeordnet, das mindestens teilweise gasdurchlässig ist und gleichzeitig als Filter dient. In beiden Fällen ist die Treibdüse der Strahlpumpe mit einer Treibgasleitung verbunden, die vorteilhafterweise mindestens über einen Teil ihrer Länge in Form einer Kapillarleitung ausgebildet sein kann.

Die mit der Erfindung erzielten Vorteile bestehen insbesondere darin, daß durch eine Verdünnung des Gas emisches mit einem Treibgas praktisch ein beliebig hoher Gemischanteil ermittelt werden kann. So können z.B. bei einer Analyse der Gasatmosphäre eines den Kernreaktor einschließenden Containments bei einer hohen Wasserstoff-Freisetzung, insbesondere auch bei einem unterstöchiometrischen Sauerstoffanteil, Wasserstoffkonzentrationen von mehr als 30 Vol.-% festgestellt werden. Dabei kann die Analyse sowohl innerhalb als auch außerhalb der die Containment-Atmosphäre einschließenden Sicherheitshülle durchgeführt werden. Weiterhin kann dieses Verfahren auch zur Ermittlung der Gemischanteile eines aus dem Primärkreis oder aus anderen druckführenden Systemen eines Kernkraftwerks abströmenden Gasgemische vorteilhaft angewendet werden. In keinem Fall wird die Zündtemperatur eines Wasserstoff-Sauerstoff-Gemisches erreicht.

Durch Verwendung unterschiedlicher Treibgase können verschiedene Gemischanteile ermittelt werden. Dabei sind eine Kalibrierung oder Umschaltung zwischen Meßbereichen einerseits, z.B. zwischen einem ersten Bereich für Gemischanteile bis 10% und einem zweiten Bereich für Gemischanteile oder Konzentrationen oberhalb von 10%, sowie Änderungen der Treibgaszusammensetzung für die Erfassung unterschiedlicher Gemischanteile andererseits innerhalb kurzer Zeit möglich.

Ausführungsbeispiele der Erfindung werden anhand einer Zeichnung näher erläutert; darin zeigen:
Figur 1 im Ausschnitt eine Sicherheitshülle eines Kernreaktors mit einer Meßeinrichtung mit einem schematisch dargestellten Meßkopf als Vorrichtung zur Erfassung von Gemischanteilen eines Gasgemisches, und
Figur 2 eine Meßeinrichtung gemäß Figur 1 mit einer Anzahl von verteilt angeordneten Verdünnungsvorrichtungen und mit in einer gemeinsamen Meßbox angeordneten Katalysatoren.

Einander entsprechende Teile sind in beiden Figuren mit den gleichen Bezugszeichen versehen.

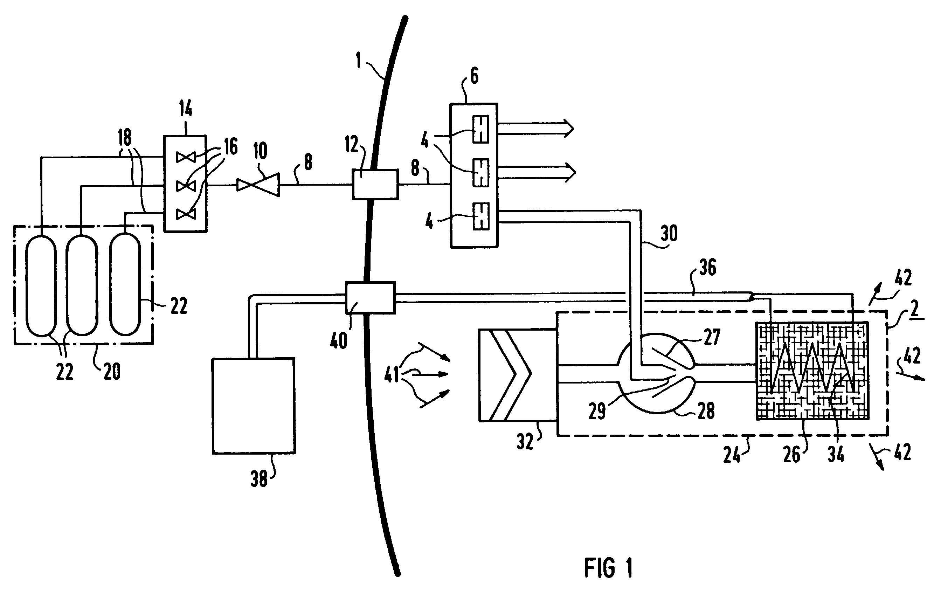

Die in Figur 1 dargestellte Meßeinrichtung umfaßt eine Anzahl von innerhalb der Sicherheitshülle 1 angeordneten Meßköpfen 2, von denen in Figur 1 nur einer schematisch dargestellt ist. Der dargestellte Meßkopf 2 sowie alle anderen Meßköpfe sind über jeweils eine Drossel 4, die in einer gemeinsamen Verteilerbox 6 angeordnet sind, mit einer Treibgasleitung 8 verbunden. Die Treibgasleitung 8, in der ein Stellglied oder Drosselventil 10 liegt, ist über eine Durchführung 12 in der Sicherheitshülle 1 mit einem Dosierblock 14 verbunden, der eine Anzahl von Ventilen 16 aufweist. Der Dosierblock 14 ist über Gasleitungen 18 mit einer Kammer 20 verbunden, in der Gasbehälter 22 aufgestellt sind. Die Gasbehälter 22 sind jeweils mit einem Treib- oder Kalibriergas gefüllt, z.B. mit Luft, Stickstoff, Wasserstoff oder Sauerstoff. Die Gasbehälter 22 sowie der Dosierblock 14 können auch innerhalb der Sicherheitshülle angeordnet sein.

Der Meßkopf 2 ist z.B. in der Art eines an sich aus der DE-OS 34 38 659 bekannten Diffusionsmeßkopfes ausgebildet. Er umfaßt ein Gehäuse 24, in dem ein Katalysator 26 und eine Strahlpumpe 28, z.B. eine Venturidüse, angeordnet sind. Anstelle der Strahlpumpe 28 kann auch eine andere Mischeinrichtung, z.B. ein statischer Mischer, vorgesehen sein. Das Gehäuse 24 ist ganz oder teilweise aus einem gesinterten Metall aufgebaut. Es ist dann aufgrund der Porosität des Sintermetalls gasdurchlässig, wobei Aerosole und Feuchtigkeit zurückgehalten werden.

Die Strahlpumpe 28 ist eingangsseitig über eine Kapillarleitung 30 mit der Verteilerbox 6 verbunden und mündet in den Katalysator 26. Sie steht außerdem eingangsseitig über ein Filter 32 mit dem durch die Sicherheitshülle 1 gebildeten Innenraum, d.h. mit der den (nicht gezeigten) Kernreaktor umgebenden Containment-Atmosphäre, in Verbindung.

Der Katalysator 26 enthält als katalytisch wirksamen Stoff Palladium oder Platin und ist netz- oder watteförmig ausgebildet. Der Katalysator 26 kann aber auch ganz oder teilweise spiralförmig in Form eines beheizbaren Filaments ausgebildet sein. Dem Katalysator 26 kann über die Treibgasleitung 8 ein Regenerationsgas zugeführt werden. Dadurch ist eine Regeneration des Katalysatormaterials auch bei Temperaturen oberhalb von 500° C möglich.

Zur Ermittlung von Temperaturänderungen im Bereich des Katalysators 26 ist ein Temperatursensor 34 vorgesehen, der über eine Meßleitung 36 mit einer Einrichtung 38 zur Meßwertaufbereitung, z.B. in einer Leitwarte, verbunden ist. Die Meßleitung 36 ist über eine Durchführung 40 in der Sicherheitshülle 1 geführt. Als Temperatursensor 34 eignet sich besonders ein einer Brückenschaltung zugeordneter temperaturabhängiger elektrischer Widerstand. Zum gleichen Zweck kann aber auch ein Thermoelement oder eine Spule mit einer temperaturabhängigen Induktivität verwendet werden.

Die in der Sicherheitshülle 1 eingeschlossene Containment-Atmosphäre ist ein Gasgemisch, das unter bestimmten Betriebsbedingungen Wasserstoff, Kohlenmonoxid, Sauerstoff und/oder Kohlenwasserstoff als Gemischanteile enthält. Zur Ermittlung eines Gemischanteils wird ein Treibgas aus einem der Behälter 22 über die Treibgasleitung 8 und über die Kapillarleitung 30 der Strahlpumpe 28 zugeführt. Die Treibgasmenge wird mittels des Stellglieds 10 eingestellt, wobei die Einstellung zweckmäßigerweise in Abhängigkeit vom Druck oder von der Temperatur des Gasgemisches sowie in Abhängigkeit von der Konzentration eines Gemischanteils des Gasgemisches, insbesondere in Abhängigkeit von der Wasserstoffkonzentration, automatisch erfolgt. Im Stellglied 10 erfolgt eine weitgehend volumenkonstante Drosselung der Treibgasmenge. Dabei erreicht das Treibgas bei einem Druckverhältnis von 1/2 zwischen dem Gasgemischdruck innerhalb der Sicherheitshülle 1 und dem Treibgasdruck Lavalgeschwindigkeit.

Eine Verteilung des Treibgases auf die einzelnen Meßköpfe 2 erfolgt in der Verteilerbox 6.

Das in Richtung der Pfeile 41 über das Filter 32 in die Strahlpumpe 28 einströmende Gasgemisch wird in einer Fangdüse 27 der Strahlpumpe 28 mit dem aus einer Treibdüse 29 der Strahlpumpe 28 mit großer Geschwindigkeit austretenden Treibgasstrahl gemischt. Dabei kann das Treibgas bei genügend hohem Treibdruck des zu messenden Gasgemisches, z.B. infolge einer Druckerhöhung innerhalb der Sicherheitshülle 1, von diesem angesaugt werden. Das somit mit dem Treibgas, dessen Zusammensetzung bekannt ist, verdünnte Gasgemisch strömt dem Katalysator 26 zu und tritt in Richtung der Pfeile 42 aus dem Meßkopf 2 aus. Die Verdünnung wird mittels des Stellglieds 10 und/oder in der Verteilerbox 6 eingestellt, wobei das Verhältnis z.B. 1 : 4 beträgt.

Am Katalysator 26 findet die katalytische Oxidation statt, wobei die Reaktionswärme oder Temperaturänderung mittels des Temperatursensors 34 erfaßt wird. Die Temperaturänderung wird in ein entsprechendes Spannungssignal umgewandelt. In der Einrichtung 38 wird aus dem Betrag des Spannungssignals oder einer Änderung in bezug auf ein Referenzsignal unter Berücksichtigung des Verdünnungsverhältnisses der Gemischanteil, z.B. die Wasserstoff- oder Sauerstoffkonzentration in der Containment-Atmosphäre, ermittelt.

Als Treibgas wird zur Ermittlung der Wasserstoffkonzentration Stickstoff oder, wenn nicht genügend Sauerstoff in der Containment-Atmosphäre vorhanden ist, Luft oder mit Sauerstoff angereicherte Luft verwendet. Zur Ermittlung des Sauerstoffanteils wird bei nur geringer Wasserstoff-Freisetzung dem Treibgas als oxidierbarer Anteil Kohlenmonoxid oder Wasserstoff zugemischt.

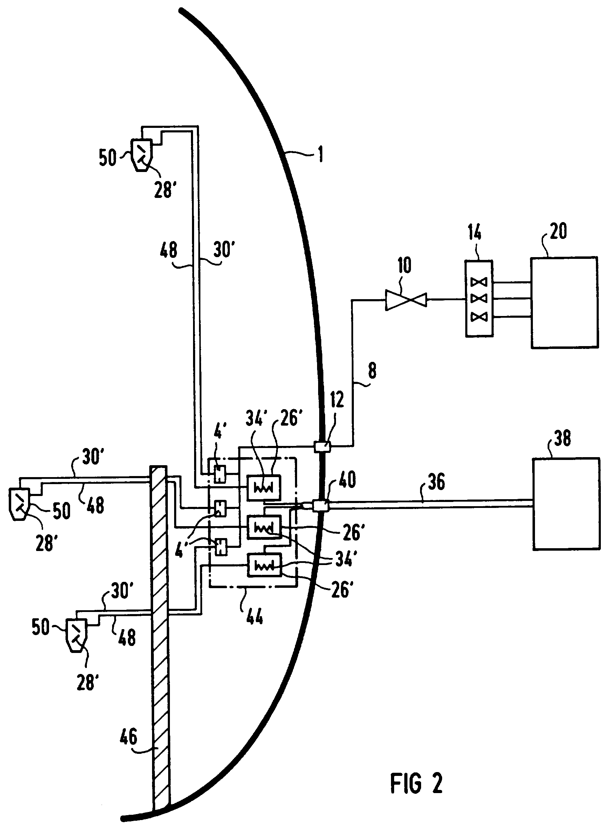

Bei der in Figur 2 dargestellten Meßvorrichtung sind mehrere Katalysatoren 26' in einer zentralen Meßbox 44 zusammengefaßt, die innerhalb der Sicherheitshülle 1 durch eine Wand 46, z.B. gegen Trümmerflug, geschützt ist. Über Zu- und Abströmleitungen 30' bzw. 48 mit der Meßbox 44 verbundene Verdünnungsvorrichtungen oder Strahlpumpen 28' sind innerhalb der Sicherheitshülle 1 verteilt angeordnet. Die in Figur 2 nur schematisch dargestellten Strahlpumpen 28' sind jeweils von einem gleichzeitig als Filter dienenden Gehäuse 50 umgeben. Jedes Gehäuse 50 besteht mindestens teilweise aus einem gasdurchlässigen Material, z.B. aus einem Sintermetall oder einem Metallfasergeflecht. Dadurch wird vermieden, daß flüssige Bestandteile oder grobe Verunreinigung mit dem Gasgemisch in die Strahlpumpen 28' gelangen.

Das über die Treibgasleitung 8 geführte Treibgas wird den Strahlpumpen 28' über die Zuströmleitungen 30' zugeführt. Die in den Zuströmleitungen 30' liegenden und innerhalb der Meßbox 44 angeordneten Drosseln 4' dienen wiederum zur Verteilung des Treibgases auf die einzelnen Strahlpumpen 28'.

Das Gasgemisch gelangt über die als Filter dienenden Gehäuse 50 in die jeweiligen Strahlpumpen 28' und wird mit dem über die Zuströmleitungen 30' strömenden Treibgas vermischt. Dabei wird das Gasgemisch gleichzeitig getrocknet, so daß eine Kondensation von im Gasgemisch enthaltener Restfeuchtigkeit, z.B. in kühlen Raumbereichen innerhalb der Sicherheitshülle 1, sicher vermieden ist. Das so verdünnte Gasgemisch gelangt über die Abströmleitungen 48 an den jeweiligen Katalysator 26'. Die Temperaturänderung jedes Katalysators 26' wird mittels eines Temperatursensors 34' getrennt gemessen. Die entsprechenden Meßsignale werden über die Leitung 36 an die Einrichtung 38 zur Meßwertaufbereitung geleitet.

Bei der Ausführungsform gemäß Figur 2 ist die Störanfälligkeit der innerhalb der Sicherheitshülle 1 verteilt angeordneten und im wesentlichen jeweils nur aus einer Strahlpumpe 28' bestehenden Bau- oder Meßgruppen besonders gering.

## Patentansprüche

1. Verfahren zur Ermittlung eines Gemischanteils eines Gasgemisches in der Containment-Atmosphäre eines Kernkraftwerks, durch Messung der bei einer katalytischen Reaktion entstehenden Temperaturänderung,
**dadurch gekennzeichnet,**
daß das dem Katalysator zugeführte Gasgemisch mit einem Treibgas bekannter Zusammensetzung verdünnt wird, wobei die Verdünnung in einer Strahlpumpe (28, 28') erfolgt.

2. Verfahren nach Anspruch 1,
**dadurch gekennzeichnet,** daß die Verdünnung einstellbar ist, vorzugsweise im Verhältnis 1 : 1 bis 1 : 10.

3. Verfahren nach Anspruch 1 oder 2,
**dadurch gekennzeichnet,** daß als Treibgas inertes Gas, vorzugsweise Stickstoff, verwendet wird.

4. Verfahren nach einem der Ansprüche 1 bis 3,
**dadurch gekennzeichnet,** daß das Treibgas einen oxidierenden Anteil, vorzugsweise Sauerstoff, enthält.

5. Verfahren nach einem der Ansprüche 1 bis 3,
**dadurch gekennzeichnet,** daß das Treibgas einen oxidierbaren Anteil, vorzugsweise Wasserstoff oder Kohlenmonoxid, enthält.

6. Verfahren nach einem der Ansprüche 1 bis 5,
**dadurch gekennzeichnet,** daß der Katalysator beheizt wird.

7. Vorrichtung zur Durchführung des Verfahrens nach einem der Ansprüche 1 bis 6 mit Mitteln (34, 34') zur Messung der bei einer katalytischen Reaktion entstehenden Temperaturänderung,
**dadurch gekennzeichnet,** daß zur Verdünnung des dem Katalysator (26, 26') zugeführten Gasgemisches mit einem Treibgas bekannten Zusammensetzung eine eingangsseitig mit einer Treibgasleitung (8, 30, 30') und ausgangsseitig mit dem Katalysator (26') verbundene Strahlpumpe (28, 28') vorgesehen ist.

8. Vorrichtung nach Anspruch 7,
**dadurch gekennzeichnet**, daß die Strahlpumpe (28) direkt in den Katalysator (26) mündendet, wobei ihr ein Filter (32) vorgeschaltet ist.

9. Vorrichtung nach Anspruch 7,
**dadurch gekennzeichnet**, daß die Strahlpumpe (28') in einem mindestens teilweise gasdurchlässigen Gehäuse (50) angeordnet und über eine Abströmleitung (48) mit dem Katalysator (26') verbunden ist.

## Claims

1. Method for determining a fraction of a gas mixture, in the containment atmosphere of a nuclear power station, by measuring the temperature change arising in a catalytic reaction, characterized in that the gas mixture fed to the catalyst is diluted with a motive gas of known composition, the dilution being effected in a jet pump (28, 28').

2. Method according to Claim 1, characterized in that the dilution is adjustable, preferably in the ratio of from 1:1 to 1:10.

3. Method according to Claim 1 or 2, characterized in that the motive gas used is inert gas, preferably nitrogen.

4. Method according to any one of Claims 1 to 3, characterized in that the motive gas contains an oxidizing fraction, preferably oxygen.

5. Method according to any one of Claims 1 to 3, characterized in that the motive gas contains an oxidizable fraction, preferably hydrogen or carbon monoxide.

6. Method according to any one of Claims 1 to 5, characterized in that the catalyst is heated.

7. Device for implementing the method according to any one of Claims 1 to 6, having means (34, 34') for measuring the temperature change arising in a catalytic reaction, characterized in that there is provided, for diluting the gas mixture fed to the catalyst (26, 26') with a motive gas of known composition, a jet pump (28, 28') which on the input side is connected to a motive gas line (8, 30, 30') and on the output side is connected to the catalyst (26').

8. Device according to Claim 7, characterized in that the jet pump (28) debouches directly into the catalyst (26), a filter (32) being connected upstream of the jet pump.

9. Device according to Claim 7, characterized in that the jet pump (28') is disposed in an at least partially gas-permeable housing (50) and via an outflow line (48) is connected to the catalyst (26').

## Revendications

1. Procédé de détermination de la proportion d'un constituant d'un mélange gazeux dans l'atmosphère de confinement d'une centrale nucléaire,en mesurant la variation de température ayant lieu lors d'une réaction catalytique,
caractérisé en ce qu'il consiste à diluer le mélange gazeux envoyé au catalyseur par un gaz d'entraînement de composition connue, la dilution s'effectuant dans un injecteur (28, 28').

2. Procédé suivant la revendication 1,
caractérisé en ce que la dilution est réglable, de préférence dans le rapport 1 : 1 à 1 : 10.

3. Procédé suivant la revendication 1 ou 2,
caractérisé en ce qu'il consiste à utiliser comme gaz d'entraînement du gaz inerte, de préférence de l'azote.

4. Procédé suivant l'une des revendications 1 à 3,
caractérisé en ce que le gaz d'entraînement renferme en partie un oxydant, de préférence de l'oxygène.

5. Procédé suivant l'une des revendications 1 à 3,
caractérisé en ce que le gaz d'entraînement renferme en partie un constituant qui peut être oxydé, de préférence de l'hydrogène ou du monoxyde de carbone.

6. Procédé suivant l'une des revendications 1 à 5,
caractérisé en ce qu'il consiste à chauffer le catalyseur.

7. Dispositif pour la mise en oeuvre du procédé suivant l'une des revendications 1 à 6, comprenant des moyens (34, 34') de mesure de la réaction de température ayant lieu lors d'une réaction catalytique,
caractérisé en ce qu'il est prévu, pour la dilution par un gaz d'entraînement de composition connue du mélange gazeux envoyé au catalyseur (26, 26'), un injecteur (28, 28') communiquant du côté de l'entrée avec un conduit (8, 30, 30') pour le gaz d'entraînement et du côté de la sortie avec le catalyseur (26').

8. Dispositif suivant la revendication 7,
caractérisé en ce que l'injecteur (28) débouche directement dans le catalyseur (26), un filtre (32) étant monté en aval de l'injecteur.

9. Dispositif suivant la revendication 7,
caractérisé en ce que l'injecteur (28') est relié au catalyseur (26') par un conduit (48) de sortie et est disposé dans une enveloppe (50) au moins partiellement perméable aux gaz.
